# EUROPEAN PATENT APPLICATION

(11) **EP 2 666 466 A1**
(43) Date of publication of application: **27.11.2013**
(21) Application number: 12736084.0
(22) Date of filing: 17.01.2012
(51) Int. Cl.: A61K 35/14, A61K 35/28, A61P 31/10, A61P 31/12, A61P 35/00, A61P 37/04, C12N 5/0783

(54) **PROCESS FOR PRODUCTION OF NK-CELL-ENRICHED BLOOD PREPARATION**

(30) Priority: 21.01.2011 JP 2011010791
(71) Applicant: Biotherapy Institute Of Japan, Tokyo 135-0051 (JP)
(72) Inventor: DENG Xuewen, Chengdu 610021 (CN); TERUNUMA Hiroshi, Tokyo 113-0033 (JP); NIEDA Mie, Tokyo 150-0013 (JP)
(74) Representative: Dick, Alexander
(86) International application number: PCT/JP2012/050789
(87) International publication number: WO 2012/099093

(57) **Abstract**

It is intended to provide a method for producing an NK cell-enriched blood preparation, which is low invasive and is capable of conveniently and rapidly growing NK cells, etc. in blood collected from an organism. The NK cells in blood are stimulated with NK cell growth-stimulating factors comprising an anti-CD16 antibody, OK432, an anti-CD3 antibody, and a cytokine. Then, the blood is cultured at a physiological cell temperature to produce an NK cell-enriched blood preparation.

## Description

### Technical Field

The present invention relates to a method for producing a blood preparation containing activated and grown NK cells, a blood preparation produced by the method, and a composition for NK cell activation.

### Background Art

Cancer, also known as malignant neoplasm, has been the leading cause of death in Japanese since 1981 and accounts for approximately 30% of all deaths. Although medical advances have drastically improved its cure rate and survival rate, cancer is still an intractable disease. For cancer, surgical therapy, chemotherapy, and radiotherapy are standard treatment methods. In recent years, immunotherapy has received attention as a novel treatment method (Non Patent Literature 1).

The immunotherapy refers to a method for treating cancer, viral infection, or the like by fortifying body's immunity. Particularly, for cancer, the immunotherapy is regarded as a new treatment method comparable to surgical therapy, chemotherapy, and radiotherapy, and various methods have been developed so far. Examples thereof include cytokine therapy, vaccinotherapy, BRM (biological response modifier) therapy, and cellular immunotherapy.

The cytokine therapy refers to a treatment method involving directly administering, into an organism, cytokines having the effect of growing or activating lymphocytes such as T cells or NK cells to thereby kill cancer cells or virus-infected cells. This corresponds to, for example, cytokine therapy based on the administration of interleukin 2 (hereinafter, referred to as "IL-2") (Non Patent Literature 2). Unfortunately, this therapy has failed to produce expected outcomes in clinical trials and causes undesired serious adverse reaction such as organ dysfunction or fluid retention (in the case of IL-2 administration), or cold symptoms or mental disorder (in the case of interferon administration).

The vaccinotherapy refers to a treatment method involving directly inoculating a human body with a cancer cell-specific antigen or the like to activate the immune system against the antigen (Non Patent Literature 3). This treatment method has been reported to be effective for some cases, but is disadvantageously ineffective for tumor or the like without HLA class I expressed therein.

The BRM therapy refers to a treatment method using a substance modifying the biological response of patients to tumor cells or the like (Non Patent Literature 4). For example, PSK, bestatin, and OK-432 are known as BRM. This treatment method, albeit with proven efficacy on some cancers, etc., is more likely to be a supportive therapy that produces effects when used in combination with surgical therapy or other treatment methods such as chemotherapy, which lowers immunity. In addition, this treatment method does not always fortify immunity and, unfortunately, its own anticancer effect or the like is weak.

The cellular immunotherapy refers to a treatment method involving subjecting immunocytes collected from a patient to *ex vivo* treatment such as activation or growth and then bringing these cells back to patient's body to enhance the immunity of the patient, and is also called "adoptive immunotherapy (adoptive immunotherapy in the broad sense)" (Non Patent Literature 5). The cellular immunotherapy is classified into activated lymphocyte therapy and dendritic cell therapy depending on the type of immunocytes treated *ex vivo.* Of them, the dendritic cell therapy has just entered the clinical stage and thus, has not yet produced sufficient results in clinical trials to determine its efficacy.

The activated lymphocyte therapy is further classified into: activated lymphocyte therapy in the narrow sense, which involves activating or growing T cells *ex vivo* (activated T lymphocyte therapy or adoptive immunotherapy in the narrow sense); and activated NK cell therapy, which involves activating or growing NK cells.

The activated lymphocyte therapy in the narrow sense corresponds to, for example, LAK (lymphokine-activated killer cell) therapy, TIL (tumor-infiltrating lymphocyte) therapy, and CTL (cytotoxic T lymphocytes) therapy.

The LAK therapy refers to a method involving adding a large amount of IL-2 to lymphocytes collected from a patient, activating or growing T cells or NK cells by culture, and then bringing these cells back to patient's body (Non Patent Literature 6). This method requires administering a large amount of IL-2 into an organism for maintaining the LAK level administered into the organism, resulting in undesired adverse reaction, as in the IL-2-based cytokine therapy, or less-than-expected effects.

The TIL therapy refers to a method involving collecting lymphocytes infiltrated into tumor cells or the like, culturing them *ex vivo* as in the LAK therapy, and then bringing them back to the body (Non Patent Literature 7). Unfortunately, for this method, surgery is only way to collect lymphocytes, and this method produces less-than-expected effects.

The CTL therapy refers to a method involving stimulating lymphocytes by coculture with cancer cells or the like collected by surgery to induce lymphocytes specific for the cancer cells or the like (Non Patent Literature 8). This method has been reported to be effective for some cases, but is highly invasive and applicable to only limited cases because cancer cells must be collected by surgery. The further problems thereof are, for example: treatment is difficult to achieve if cancer cells can be neither collected nor cultured; and this method is effective only for cancer expressing major histocompatibility antigens.

Meanwhile, the activated NK cell therapy refers to a method involving introducing grown and activated NK cells into the body. NK cells are a population of lymphocytes capable of killing cancer cells or virus-infected cells without being sensitized to antigens (Non Patent Literatures 9 to 11). The NK cells are known to be capable of suppressing cancer infiltration or metastasis in animal experiments (Non Patent Literature 12). According to long-term large-scale cohort study, it has been reported that cancer occurs with a significantly low incidence in humans having highly active NK cells in peripheral blood (Non Patent Literature 13). Hence, NK cells from a patient can be grown and activated in large amounts *ex vivo* and then brought back into patient's body to thereby treat cancer, viral infection, or the like in the patient. The activated NK cell therapy is a treatment method based on such principles. In general, NK cells are found to make up only a small percent to a dozen percent of lymphocytes even in healthy individuals, and the number of NK cells is further reduced in the case of cancer patients. Moreover, NK cells in blood often exhibit lower cytotoxic activity against cancer cells in cancer patients than in healthy individuals even when the same numbers of NK cells are present therein. Thus, growth and activation by culture are absolutely necessary for the therapy. NK cells had been considered difficult to grow *ex vivo.* Nevertheless, many studies have reported in recent years that NK cells were successfully grown and cultured (Non Patent Literatures 14 to 17). These methods, however, utilize cancer cells cultured for NK cell growth or activation or transformed cells and thus, have not yet overcome problems associated with safety in clinical application or practicality. Also, NK cell growth efficiency and cell activity have been at the less-than-satisfactory level.

As described above, all the conventional immunotherapy methods have presented insufficient therapeutic effects, serious adverse reaction, or other possible improvements thereto.

Thus, as a result of conducting diligent studies to solve these problems, the inventors of the present application had successfully developed a method for producing an NK cell-enriched blood preparation, which is capable of efficiently growing and activating NK cells in blood collected from an organism, and received a patent for the production method and the NK cell-enriched blood preparation (Patent Literature 1). The NK cell-enriched blood preparation obtained by this method has been used actually in the clinical stage to produce a large number of very favorable clinical outcomes (Non Patent Literatures 18 to 20). This production method, however, has a slightly complicated production process in which a medium must be kept at a particular temperature for a relatively long time (10 to 30 hours) for the sufficient activation of NK cells. In addition, this method requires laborious temperature control and much time to complete the preparation.

### Citation List

### Patent Literature

Patent Literature 1: JP Patent No. 4275680

### Non Patent Literature

Non Patent Literature 1: Milani V, et al., 2009, J trans Res, 7 (50): 1-18.
Non Patent Literature 2: Rosenberg SA, et al., 1985, J Exp Med., 161: 1169-88.
Non Patent Literature 3: Bendandi, M. et al., 1999, Nature Med, 5: 1171-1177.
Non Patent Literature 4: Fisher M, et al., 2002, Anticancer Res., 22: 1737-54.
Non Patent Literature 5: Takayama Y et al., 2000, Lancet, 356: 802-807.
Non Patent Literature 6: Mule JJ, et al., 1985, J Immunol., 135: 646-52.
Non Patent Literature 7: Dudley ME, et al., 2003, J Immunother., 26: 332-42.
Non Patent Literature 8: Araki K et al., 2000, Int J Oncol., 17 (6): 1107-18.
Non Patent Literature 9: Stagg J and Smyth M J., 2007, Drug News Perspect, 20 (3): 155-163.
Non Patent Literature 10: Terme M et al., 2008 Nat. Immunol, 9 (5): 486-493.
Non Patent Literature 11: Vivier E et al., 2008, Nat. Immunol, 9 (5): 503-510.
Non Patent Literature 12: Dewan MZ et al., 2007, Breast Cancer Res Treat, 104: 267-275.
Non Patent Literature 13: Imai K et al., 2000, Lancet, 356: 1795-1799.
Non Patent Literature 14: Harada H et al., 2002, JPN J Cancer Res, 93: 313-9.
Non Patent Literature 15: Carlens S et al., 2001 Hum Immunol, 62: 1092-8.
Non Patent Literature 16: Berg M et al., 2009, Cytotherapy, 11 (3): 341-55.
Non Patent Literature 17: Fujisaki H et al., 2009, Cancer Res, 9: 4010-7.
Non Patent Literature 18: Brillard E et al., 2007, Exp Hemato, 35: 416-425.
Non Patent Literature 19: Cooke A and Brode S., 2008, Critical Rev Immunol., 28 (2): 109-126.
Non Patent Literature 20: Hsu KC et al., 2005, Blood, 105: 4878-4884.

### Summary of Invention

### Technical Problem

An object of the present invention is to develop a novel method for producing an NK cell-enriched blood preparation, which is low invasive to donors and patients and is capable of rapidly growing and activating NK cells in blood collected from an organism in large amounts by a convenient production process, and to provide an NK cell-enriched blood preparation obtained by the method in a safe and relatively inexpensive manner.

### Solution to Problem

In order to attain the object, the present inventors have conducted further studies on a method for producing an NK cell-enriched blood preparation, and consequently successfully developed a novel method which does not involve, as an necessary requirement, an essential activation step comprising keeping cells at a particular temperature for a particular time in the production method according to the prior application (Japanese Patent Application No. 2006-124630; JP Patent No. 4275680) (hereinafter, referred to as the "prior application" in the present specification) for the patented invention of the present inventors, and which is capable of producing an NK cell-enriched blood preparation with higher NK cell growth activity than that in the production method according to the prior application.

The present invention has been completed on the basis of these results and provides the followings:

(1) A method for producing an NK cell-enriched blood preparation, comprising:
   a stimulation step of stimulating NK cells contained in blood collected from an organism, with NK cell growth-stimulating factors comprising at least an anti-CD 16 antibody, OK432, an anti-CD3 antibody, and a cytokine; and a culture step of culturing the blood at a physiological cell temperature after the stimulation step.

(2) The production method according to (1), wherein the stimulation step further comprises keeping the NK cells at 38°C to 40°C for 10 hours to 30 hours to apply thereto high-temperature stimulation.

(3) The production method according to (1) or (2), wherein the anti-CD3 antibody is muromonab-CD3.

(4) The production method according to any of (1) to (3), wherein the cytokine is IL-2.

(5) The production method according to any of (1) to (4), wherein the NK cell growth-stimulating factors further comprise a bisphosphonate derivative or a salt thereof, or a hydrate thereof.

(6) The production method according to (5), wherein the bisphosphonate derivative is selected from the group consisting of zoledronic acid, pamidronic acid, alendronic acid, risedronic acid, ibandronic acid, incadronic acid, etidronic acid, and a combination thereof.

(7) The production method according to any of (1) to (6), wherein the anti-CD16 antibody is immobilized on a solid-phase support.

(8) The production method according to any of (1) to (7), wherein the culture period in the culture step is 7 days to 21 days.

(9) The production method according to any of (1) to (8), wherein the blood is peripheral blood, cord blood, or bone marrow fluid.

(10) An NK cell-enriched blood preparation obtained by a production method according to any of (1) to (9).

(11) A composition for NK cell enrichment comprising an anti-CD16 antibody, OK432, an anti-CD3 antibody, and a cytokine.

(12) The composition according to (11), wherein the anti-CD3 antibody is muromonab-CD3.

(13) The composition according to (11) or (12), wherein the cytokine is IL-2.

(14) The composition according to any of (11) to (13), further comprising a bisphosphonate derivative or a salt thereof, or a hydrate thereof.

(15) The composition according to (14), wherein the bisphosphonate derivative is selected from the group consisting of zoledronic acid, pamidronic acid, alendronic acid, risedronic acid, ibandronic acid, incadronic acid, etidronic acid, and a combination thereof.

(16) A kit for production of NK cell-enriched blood comprising a composition for NK cell enrichment according to any of (11) to (15).

The present specification encompasses the contents described in the specification and/or drawings of Japanese Patent Application No. 2011-010791 which serves as a basis for the priority of the present application.

### Advantageous Effects of Invention

The method for producing an NK cell-enriched blood preparation according to the present invention can prepare NK cells in blood more rapidly and more conveniently at a more improved growth rate than conventional methods. Moreover, the production method of the present invention is capable of production from peripheral blood and is thus advantageously low invasive to donors and patients.

The method for producing an NK cell-enriched blood preparation according to the present invention further using a bisphosphonate derivative as one NK cell growth-stimulating factor can enhance not only the growth of NK cells but also the growth of γδT cells. As a result, a blood preparation more effective for cellular immunotherapy can be provided.

### Brief Description of Drawings

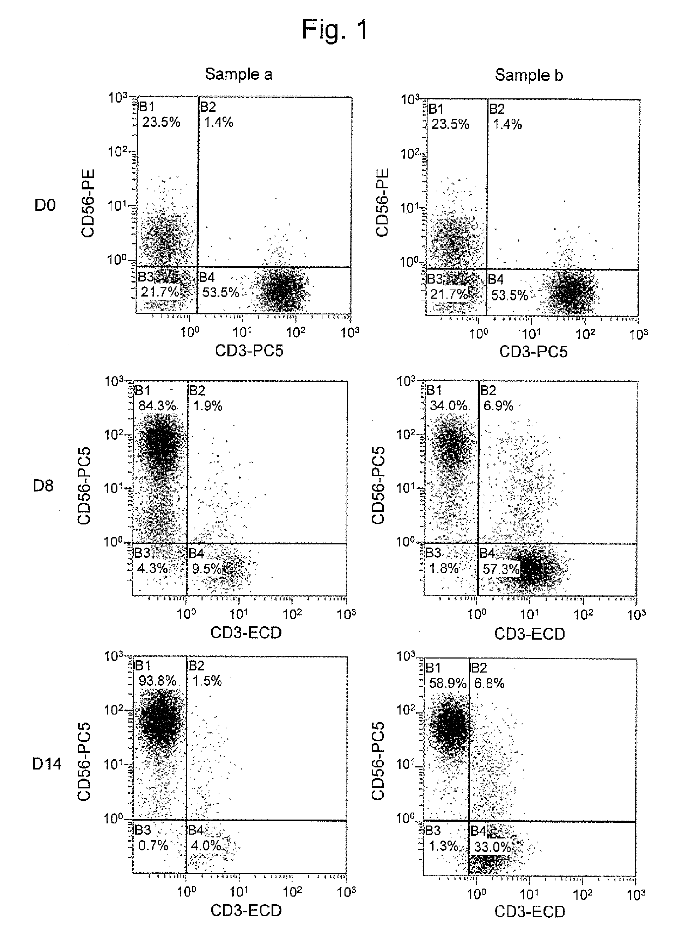

[Figure 1] Figure 1 shows a cytogram at day 0 (D0), day 8 (D8) and day 14 (D14) after the start of culture for samples a and b of donor #2. In each cytogram, the abscissa represents the fluorescence intensity of a PC5-labeled CD3 antibody (D0) or an ECD-labeled anti-CD3 antibody (D8 and D14) on a log scale. The ordinate represents the fluorescence intensity of a PE-labeled anti-CD56 antibody (D0) or a PC5-labeled anti-CD56 antibody (D8 and D14) on a log scale. The cytogram is divided into four zones (B1 to B4) based on these various fluorescence intensities. NK cells are distributed in zone B1 (CD3-CD56⁺); T lymphocytes are distributed in zones B2 (CD3⁺CD56⁺) and B4 (CD3⁺CD56⁻); and the other cells, such as B cells, are distributed in zone B3 (CD3⁻CD56⁻). The numeric value in each fraction represents the ratio (%) of the cells contained in the fraction to all the assayed cultured cells.

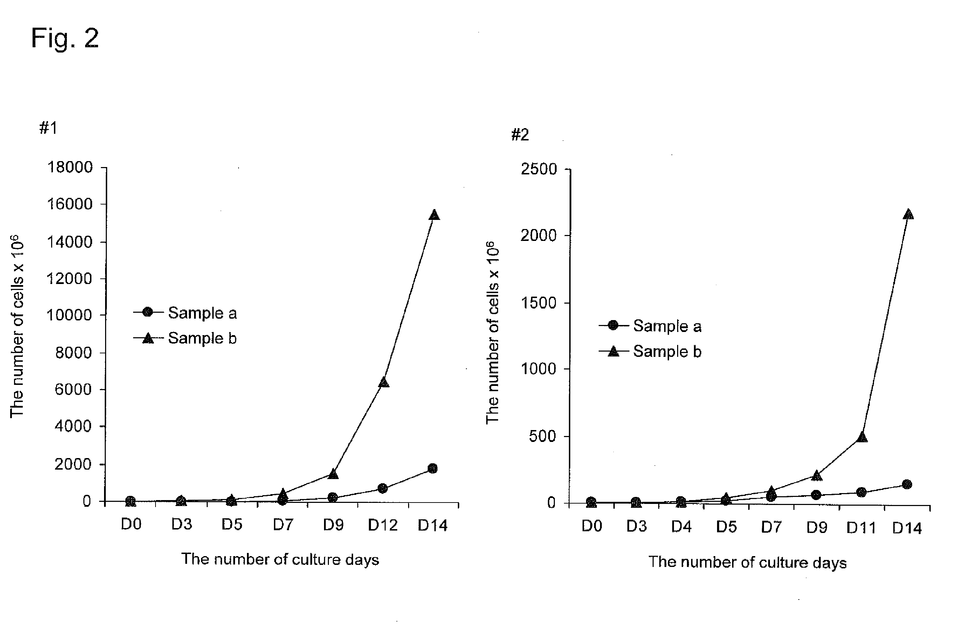

[Figure 2] Figure 2 is a cell growth curve showing the relationship between the number of culture days and the total number of cultured cells for samples a and b of donors #1 and #2. D0, D3, D5, D7, D9, D12, and D14 represent day 0, day 3, day 5, day 7, day 9, day 12, and day 14, respectively, after the start of culture.

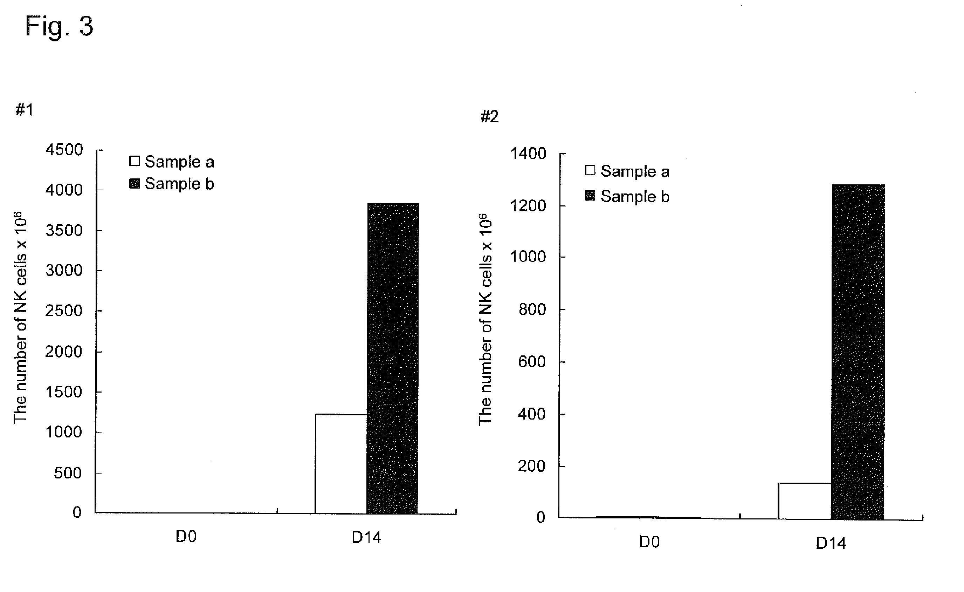

[Figure 3] Figure 3 shows the absolute number of NK cells in samples a and b of donors #1 and #2 at day 0 (D0) and day 14 (D 14) after the start of culture. The absolute number ofNK cells was calculated on the basis of the ratio of the NK cell zone (B1) of Figure 1 and the total number of cultured cells of Figure 2.

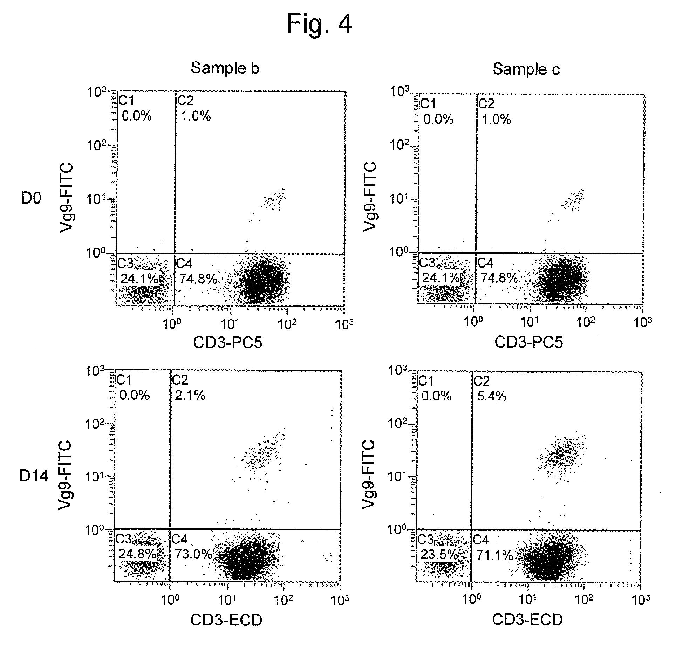

[Figure 4] Figure 4 is a cytogram showing results of assaying γδT cells at day 0 (D0) and day 14 (D14) after the start of culture for samples b and c of donor #1. In each cytogram, the abscissa represents the fluorescence intensity of a PC5-labeled anti-CD3 antibody (D0) or an ECD-labeled anti-CD3 antibody (D14) on a log scale. The ordinate represents the fluorescence intensity of an FITC-labeled anti-Vγ9 antibody on a log scale. The cytogram is divided into four zones (C1 to C4) based on these various fluorescence intensities. No cell is present in zone C1 (CD3-Vγ9⁺) as a rule; γδT cells are distributed in zone C2 (CD3⁺Vγ9⁺); cells (including NK cells) other than T cells are distributed in zone C3 (CD3⁻Vγ9⁻); and αβT cells are distributed in zone C4 (CD3⁺Vγ9⁻). The numeric value in each fraction represents the ratio (%) of the cells contained in the fraction to all the assayed cultured cells.

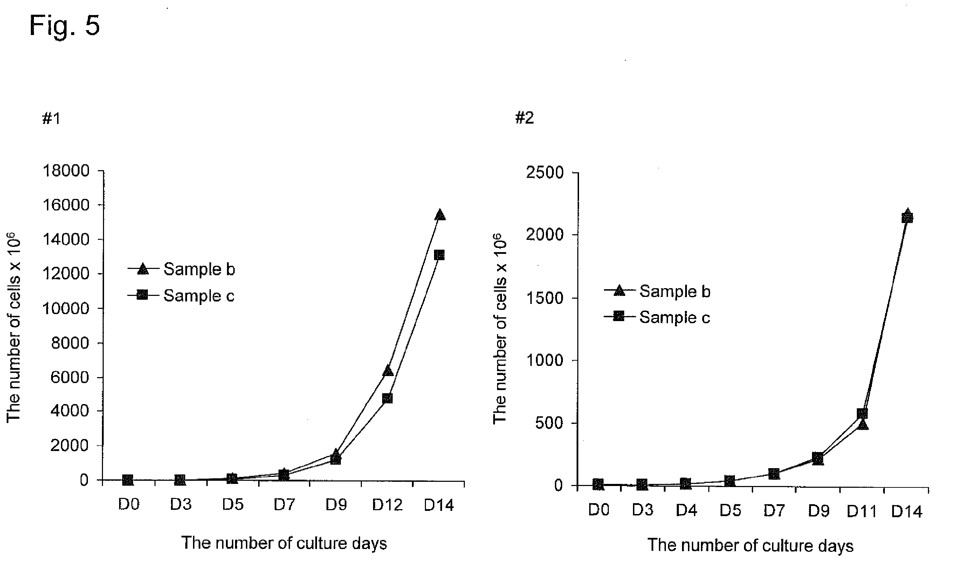

[Figure 5] Figure 5 is a cell growth curve showing the relationship between the number of culture days and the total number of cultured cells of samples b and c for donors #1 and #2.

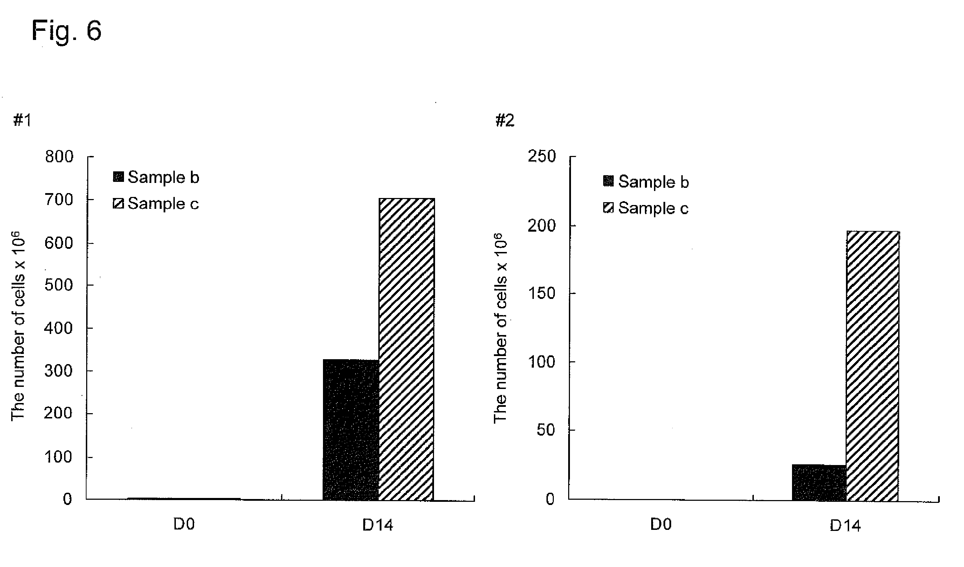

[Figure 6] Figure 6 shows the absolute number of γδT cells in samples b and c of donors #1 and #2 at day 0 (D0) and day 14 (D14) after the start of culture. The absolute number of γδT cells was calculated on the basis of the ratio of the γδT cell zone (C2) of Figure 4 and the total number of cultured cells of Figure 5.

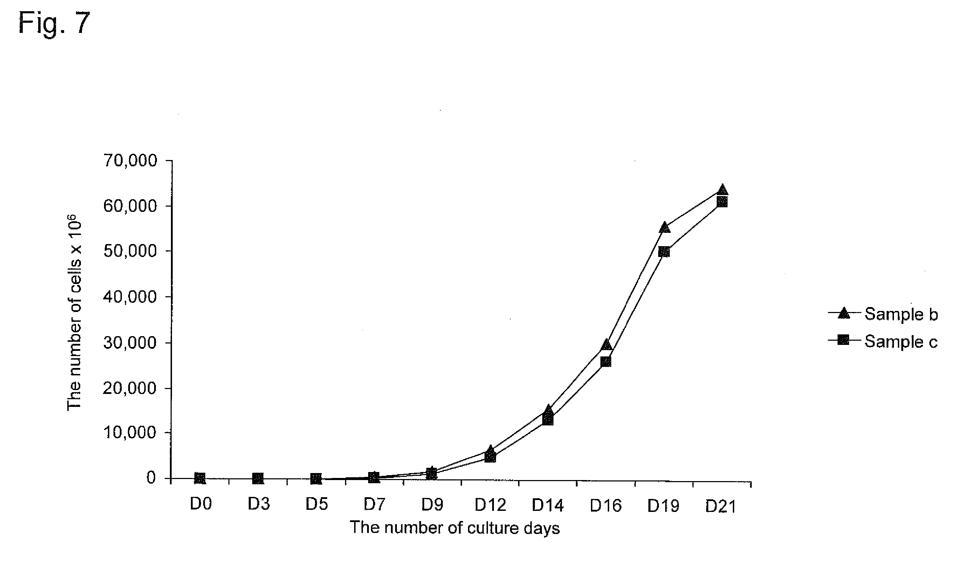

[Figure 7] Figure 7 is a cell growth curve showing the relationship between the number of culture days and the total number of cultured cells for samples b and c of donor #1. D0, D3, D5, D7, D9, D12, D14, D16, D19, and D21 represent day 0, day 3, day 5, day 7, day 9, day 12, day 14, day 16, day 19, and day 21, respectively, after the start of culture.

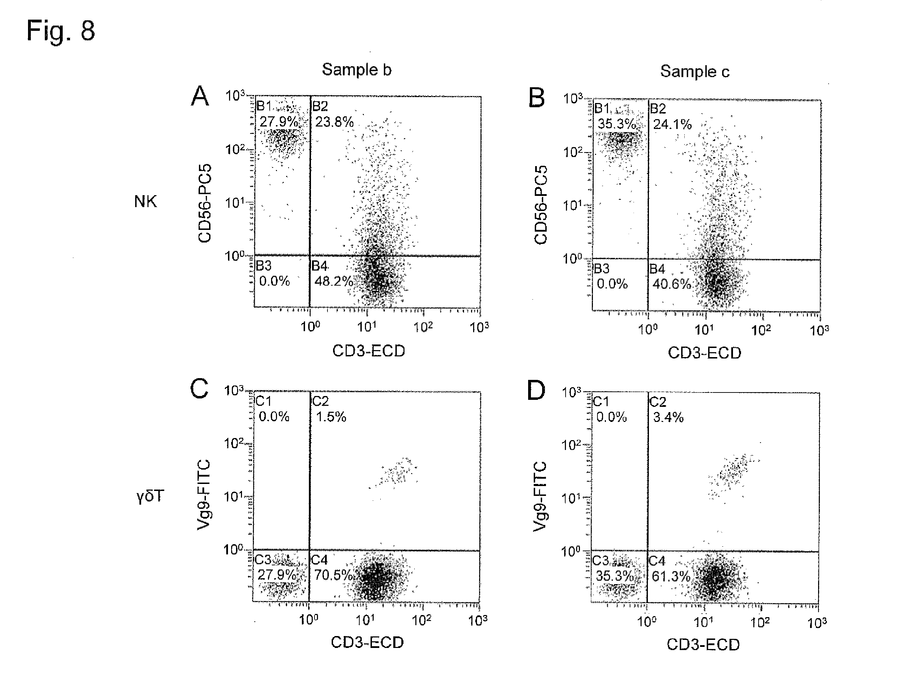

[Figure 8] Figure 8 is a cytogram showing results of assaying NK cells (Figures 8A and 8B) and γδT cells (Figures 8C and 8D) in samples b and c of donor #1 at day 21 (D21) after the start of culture. In all of Figures 8A to 8D, the abscissa represents the fluorescence intensity of an ECD-labeled anti-CD3 antibody on a log scale. In Figures 8A and 8B, the ordinate represents the fluorescence intensity of a PC5-labeled anti-CD56 antibody on a log scale. In
Figures 8C and 8D, the ordinate represents the fluorescence intensity of an FITC-labeled anti-Vy9 antibody on a log scale. Cells distributed in each zone were identified in the same way as in Figure 1 (NK cells) and Figure 4 (γδT cells).

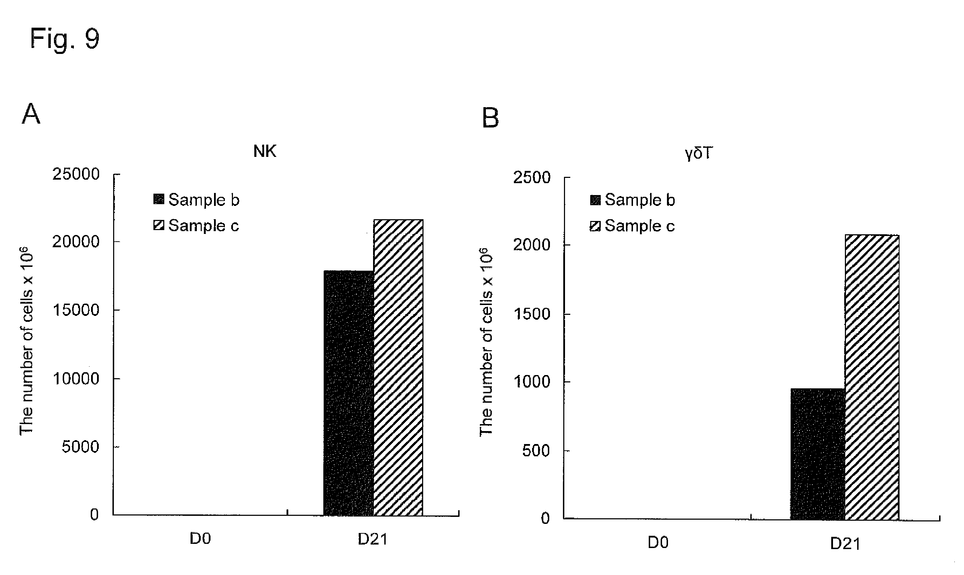

[Figure 9] Figure 9 shows the absolute numbers ofNK cells (Figure 9A) and γδT cells (Figure 9B) in samples b and c of donor #1 at day 0 (D0) and day 21 (D21) after the start of culture. The absolute number of each cell was calculated on the basis of the ratios of the NK cell zone (B1) and the γδT cell zone (C2) of Figure 8 and the total number of cultured cells of Figure 7.

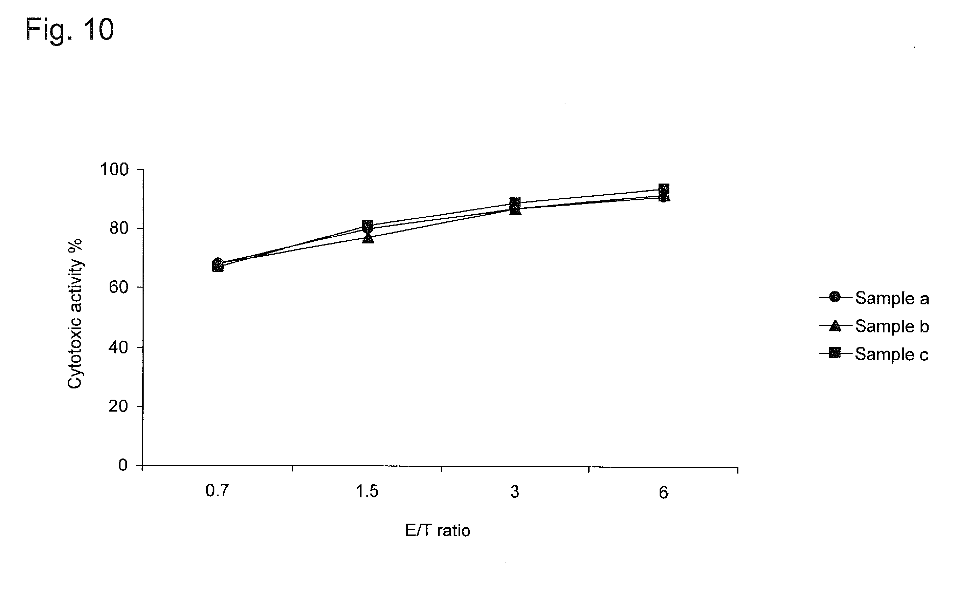

[Figure 10] Figure 10 shows the cytotoxic activity ofNK cells in samples a, b, and c produced from donor #2 by a method of Example 2. The E/T ratio shown in the X-axis is the ratio between NK cells used as effector cells (E) and target K562 cells (target cells: T).

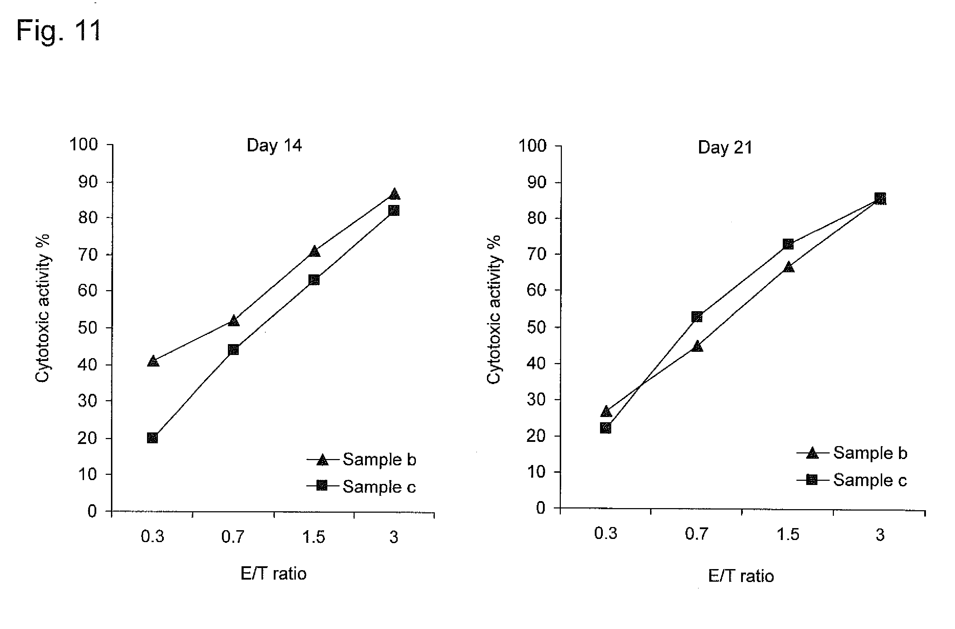

[Figure 11] Figure 11 shows the cytotoxic activity at day 14 and day 21 of NK cells in samples b and c produced from donor #1 by a method of Example 3.

### Description of Embodiments

### 1. Method for producing NK cell-enriched blood preparation

### 1-1. Summary

The first embodiment of the present invention relates to a method for producing an NK cell-enriched blood preparation. The feature of this embodiment is that NK cells in blood collected from an organism are stimulated with growth-stimulating factors, and then, the blood is cultured at a physiological cell temperature.

In the present invention, the "NK cell-enriched blood preparation" refers to a preparation mainly composed of blood containing a large number of activated NK cells obtained by the production method of this embodiment. In the present invention, the term "enrichment" means to grow and activate cells or to have grown and activated cells. In the present specification, the term "activation" means to enhance or potentiate functions possessed by cells, particularly, NK cells. Examples thereof include to potentiate cytotoxic function and to potentiate the expression of NK cell surface receptors involved in activity and/or growth.

### 1-2. Constitution

The method for producing an NK cell-enriched blood preparation according to this embodiment comprises a stimulation step and a culture step. Hereinafter, the constitution of each step will be described specifically. This embodiment is based on the premise that, as a rule, each operation employs already sterilized reagents, media, tools, etc., and culture is performed in a sterile environment such as a clean bench in a clean room. This is because blood and the NK cell-enriched blood preparation produced in this embodiment are prevented from being contaminated with bacteria or the like.

### 1-2-1. Stimulation step

The "stimulation step" is the step of stimulating NK cells contained in blood collected from an organism with NK cell growth-stimulating factors.

### (1) Blood

In the present invention, the "blood" refers to a blood component containing at least NK cells. The blood (component) corresponds to, for example, whole blood, cord blood, bone marrow fluid, or a portion of its components, for example, mononuclear cells. Of them, mononuclear cells are very preferable. This is because blood components such as erythrocytes or granulocytes might become a hindrance to the production of the NK cell-enriched blood preparation. Peripheral blood mononuclear cells (PBMCs) obtained from peripheral blood are particularly preferable. This is, for example, because peripheral blood can be collected easily from organisms at any time with procedures low invasive to the donors.

In the present invention, the "organism" refers to a living mammal. The type of the mammal is not particularly limited, and a human is preferable. The donor organism is of the same type as in an animal that receives the NK cell-enriched blood preparation obtained by the production method of the present invention. For example, when the NK cell-enriched blood preparation of the present invention is administered to a human, blood is collected from a human. Most preferably, blood is collected from an organism itself that receives the NK cell-enriched blood preparation of the present invention, i.e., blood collection is predicated on adoptive immunotherapy. This is because a blood preparation derived from recipient's own blood can minimize the possibility of rejection after administration. When blood collection is predicated on adoptive immunotherapy, the donor organism does not have to be healthy. For example, blood can be collected even from a patient having cancer or viral infection. In the present invention, the adoptive immunotherapy in the description below means the adoptive immunotherapy in the broad sense described above, unless otherwise specified.

The phrase "collected from an organism" means derived from an organism. Thus, this phrase encompasses any of blood directly collected from the organism and blood indirectly collected therefrom. The directly collected blood corresponds to, for example, peripheral blood or bone marrow fluid collected by the direct insertion of an injection needle or the like to the organism, or, for example, cord blood collected directly from the postpartum umbilical cord. Alternatively, the indirectly collected blood refers to, for example, blood obtained by adding heparin or the like for anticoagulation treatment to the directly collected blood or further isolating mononuclear cells therefrom and then temporarily refrigerating or cryopreserving it, followed by collection.

### (2) Preparation of blood

When the blood used in this step is directly collected from the organism, a collection method therefor can follow a blood collection method known in the art. For example, peripheral blood may be collected by injection to the peripheral vein or the like; bone marrow fluid may be collected by bone marrow aspiration; and cord blood may be collected by the injection of a needle to the postpartum umbilical cord before placenta delivery. Hereinafter, the collection of peripheral blood will be described specifically.

Peripheral whole blood can be collected into a syringe by the insertion of an injection needle to the peripheral blood vessel, for example, the vein or artery, of the organism. The volume of blood collected varies depending on the necessary amount of the NK cell-enriched blood preparation. Usually, 20 mL to 60 mL suffices for the blood preparation produced, for example, for a single dose to a human adult. However, the number of peripheral blood mononuclear cells in blood may be extremely reduced, for example, in cancer patients. In such a case, only peripheral blood mononuclear cells may be collected selectively in a necessary amount by apheresis. For preventing the coagulation of the blood thus collected, it is preferred to coat in advance, for example, the inside of a syringe, with an anticoagulant such as heparin or a blood coagulation inhibitor, or to add heparin or the like to the collected blood. Alternatively, plasma is separated from the peripheral whole blood, and the remaining blood cell components may be used in the present invention. The plasma can be obtained as a supernatant, for example, by the centrifugation at 2000 rpm to 4000 rpm for 5 to 20 minutes of peripheral whole blood transferred to a centrifuge tube. This supernatant can be inactivated by heating at 56°C for approximately 30 minutes, then centrifuged at 2000 rpm to 4000 rpm for 5 to 20 minutes, and also used as nutrients for cell culture after removal of precipitates such as platelet.

Peripheral blood mononuclear cells (PBMCs) may be further separated, if necessary, from the peripheral whole blood. PBMCs can be obtained from peripheral whole blood or from blood cell components after plasma separation using a density-gradient centrifugation method with Ficoll-Hypaque or Ficoll-Conray as a specific gravity solution. A commercially available separating solution or the like can be used conveniently as such a specific gravity solution. For example, Ficoll-Paque PLUS (GE Healthcare (formerly Amersham Biosciences Corp.)) or LYMPHOPREP (AXIS-SHIELD plc) can be used. A method for separating PBMCs can follow the protocol supplied with the kit.

The PBMCs thus separated are washed several times with PBS (-) or a medium for cultured cells to remove the specific gravity solution. In this context, for example, serum-free PBS (-), a RPMI1640 medium, or a serum-free medium for use in culture can be used as the medium for cultured cells. After the washing with this PBS (-) or medium, it is preferred to count the number of collected PBMCs using a hemacytometer. Usually, 2 × 10⁷ or more PBMCs can be collected from 20 mL to 60 mL of peripheral whole blood.

When the blood used in this step is indirectly collected from the organism, a collection method therefor can involve thawing or heating frozen or refrigerated blood for use by a method known in the art. Examples thereof include a method involving adding a RPMI-1640 medium, for thawing, to the PBMCs obtained from the directly collected blood and then cryopreserved, and then incubating the thawed PBMCs at 37°C for 3 hours under 5% CO² condition.

### (3) NK cell growth-stimulating factor

In the present invention, the "NK cell growth-stimulating factor" refers to a factor directly or indirectly inducing the growth and/or activation (hereinafter, referred to as "growth/activation") of NK cells. Examples of the factor directly inducing the growth include factors having the function of transmitting growth signals into NK cells through the specific binding to the surface receptors of the NK cells to be grown. Examples of the factor indirectly inducing the growth include factors inducing the production and release of liquid factors such as cytokines through the binding to the surface receptors of cells other than NK cells, such as monocytes. In this case, the growth of NK cells is indirectly induced by the released liquid factor. Examples of the factor directly or indirectly inducing the activation of NK cells include factors similar in mechanism to the factors inducing the growth.

The NK cell growth-stimulating factors of the present invention comprise at least an anti-CD16 antibody, an anti-CD3 antibody, OK432, and a cytokine.

The "anti-CD16 antibody" refers to an antibody against an antigen CD16. CD16 serves as a marker for NK cells or granulocytes and is known as a protein constituting Fc receptor present on the surfaces of all NK cells in the resting period. The NK cell growth-inducing activity of the anti-CD16 antibody was found by the prior patent application and had been unknown before then. Although the mechanism underlying the induction of NK cell growth by the anti-CD16 antibody remains to be elucidated, the co-addition of the anti-CD16 antibody and a cytokine such as IL-2 can drastically potentiate the induction of NK cell growth compared with the addition of the cytokine alone (Japanese Patent Application No. 2006-124630; JP Patent No. 4275680). This antibody can be any of monoclonal and polyclonal antibodies. A monoclonal antibody is preferable.

The "anti-CD3 antibody" refers to an antibody against CD3. The anti-CD3 antibody used as an NK cell growth-stimulating factor of the present invention is not particularly limited as long as the antibody specifically recognizes CD3 and binds thereto. This antibody can be any of monoclonal and polyclonal antibodies. A monoclonal antibody is preferable. Examples thereof include muromonab-CD3 (trade name: Orthoclone OKT3 (registered trademark), Janssen Pharmaceutical K.K.).

The "OK432" (trade name: Picibanil) refers to an antitumor agent comprising a penicillin-treated Su strain of hemolytic streptococcus (type III group A *Streptococcus pyogenes*) as an active ingredient and belongs to the BRM described above. OK432 is known to serve as an immune adjuvant capable of activating, for example, monocytes, through the binding to the surface TLR of the monocytes so that immune response is activated (Ryoma Y, et al., 2004, Anticancer Res., 24: 3295-301.).

Examples of the cytokine appropriate as an NK cell growth-stimulating factor of the present invention include IL-2, IL-12, IL-15, and IL-18. Of them, a particularly preferable cytokine is IL-2.

The NK cell growth-stimulating factors of the present invention can further comprise a bisphosphonate derivative or a salt thereof, or a hydrate thereof, and/or BRM other than OK432, etc.

The "bisphosphonate derivative" refers to a compound represented by the following general formula 1:

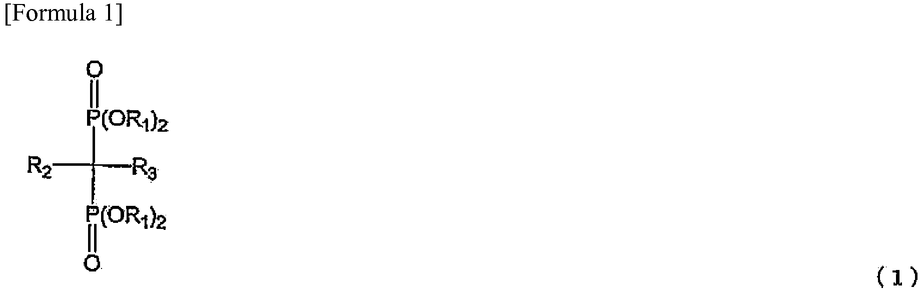

In the formula, R₁ represents a hydrogen atom (H) or a lower alkyl group; and R₂ and R₃ each independently represent a hydrogen atom, halogen, a hydroxyl group, an amino group, a thiol group, a substituted or unsubstituted aryl group, a substituted or unsubstituted alkyl group, a lower alkylamino group, an aralkyl group, a cycloalkyl group, or a heterocyclic group, or R₂ and R₃ form a portion of a cyclic structure containing them wherein substituents forming the cyclic structure are each independently derived from halogen, a lower alkyl group, a hydroxyl group, a thiol group, an amino group, an alkoxy group, an aryl group, an arylthio group, an aryloxy group, an alkylthio group, a cycloalkyl group, or a heterocyclic group in R₂ and R₃.

Specific examples of the bisphosphonate derivative include zoledronic acid, pamidronic acid, alendronic acid, risedronic acid, ibandronic acid, incadronic acid, and etidronic acid. In the present invention, one or more bisphosphonate derivatives or salts thereof, or hydrates thereof can be added as the NK cell growth-stimulating factor. In the present invention, the bisphosphonate derivative is particularly preferably zoledronic acid or a zoledronic acid derivative capable of inducing the growth/activation of NK cells or a salt thereof, or a hydrate thereof.

The zoledronic acid (trade name: Zometa (registered trademark), Novartis Pharma K.K.) is bisphosphonate having bone resorption inhibitory activity and is known as a therapeutic drug for hypercalcemia caused by malignant tumor, bone lesions attributed to multiple myeloma, and bone lesions attributed to solid cancer metastasized to bone. Since its chemical structure incorporates nitrogen-containing bisphosphonates (N-BPs), this acid inhibits the intracellular synthesis of farnesyl pyrophosphate (FPP), resulting in the accumulation of its precursor isopentenyl pyrophosphate (IPP). As a result, the immune response of the organism can reportedly be activated (van Beek E, et al., 1999, Biochem Biophys Res Commun, 264: 108-11; and Gober HJ, et al., 2003, J Exp Med, 197: 163-8.).

The "salt thereof" refers to a base-addition salt of the bisphosphonate derivative, preferably zoledronic acid. Examples of the base-addition salt include: alkali metal salts such as sodium salt and potassium salt; alkaline earth metal salts such as calcium salt and magnesium salt; aliphatic amine salts such as trimethylamine salt, triethylamine salt, dicyclohexylamine salt, ethanolamine salt, diethanolamine salt, triethanolamine salt, and procaine salt; aralkylamine salts such as N,N-dibenzylethylenediamine; heterocyclic aromatic amine salts such as pyridine salt, picoline salt, quinoline salt, and isoquinoline salt; basic amino acid salts such as arginine salt and lysine salt; and ammonium salt and quaternary ammonium salts such as tetramethylammonium salt, tetraethylammonium salt, benzyltrimethylammonium salt, benzyltriethylammonium salt, benzyltributylammonium salt, methyltrioctylammonium salt, and tetrabutylammonium salt.

The "BRM" refers to a substance that brings about therapeutic effects by modifying the biological response of hosts to tumor cells as described above. Examples thereof include protein-polysaccharide complexes extracted from basidiomycetes, more specifically, lentinan extracted from *Lentinula edodes* and krestin extracted from *Trametes versicolor.*

### (4) Method for applying stimulation

The term "stimulating" refers to contacting the NK cell growth-stimulating factors with NK cells to thereby induce the growth/activation of the NK cells.

In a specific method for applying stimulation thereto, first, blood, for example, PBMCs, collected from an organism is adjusted with a medium into, for example, a cell density of 1 to 3 × 10⁶ cells/mL. In this context, the medium used may be any appropriate medium for cell culture supplemented with inactivated human serum or plasma at a volume ratio (V/V) on the order of 5 to 10%. When the blood preparation is predicated on administration for the adoptive immunotherapy, desirably, a serum-free medium for adoptive immunotherapy such as OpTmizer supplemented with autologous plasma is used as the medium. The autologous plasma can be prepared from blood obtained after the blood collection step. For example, the collected peripheral whole blood is centrifuged at 3000 rpm at room temperature (10°C to 30°C: the same holds true for the description below) for approximately 10 minutes to obtain a supernatant, which can in turn be used as the autologous plasma. If necessary, the medium may be further supplemented with an antibiotic such as streptomycin, penicillin, kanamycin, or gentamicin.

Next, each NK cell growth-stimulating factor is added to the culture solution containing the preliminarily prepared PBMCs.

For stimulation with the anti-CD16 antibody, this antibody can be added directly to the medium at, for example, 0.01 µg/mL to 100 µg/mL, preferably 0.1 µg/mL to 10 µg/mL, more preferably 1 µg/mL, in terms of the final concentration, or can be added thereto in a form immobilized on a solid-phase support. The addition of the antibody in a form immobilized on a solid-phase support is preferable. This is because the anti-CD16 antibody thus immobilized on a solid phase can come into contact with NK cells with increased frequency in the constant direction and thus efficiently impart growth stimulation to the NK cells compared with a free form. In this context, the "support" refers to a scaffold for antibody immobilization. A material for the support is not particularly limited as long as this material permits stable immobilization of the antibody. For example, a synthetic resin (e.g., plastic), glass, or a metal can be used. The shape of the support is not particularly limited. A shape with a large surface area of contact with the culture solution is preferable because the antibody immobilized on this support can come into contact with the NK cells with higher frequency. Examples thereof include spherical beads and porous cubes having lymphocyte-sized pores.

When the support is made of a material with high affinity for the antibody, for example, plastic, the anti-CD16 antibody can be immobilized on this support by a simple method of contacting (including dipping, coating, circulating, spraying, etc.) the antibody solution with the support and keeping them at a predetermined temperature for a predetermined time. The anti-CD16 antibody solution can be obtained, for example, by dissolving the anti-CD16 antibody in sterile distilled water or a medium for cell culture, then sterilizing, if necessary, by filtration through, for example, a filter of 0.22 µm in pore size, and adjusting the filtrate with sterile distilled water or a medium to 1 µg/mL in terms of the final concentration. For immobilizing the anti-CD16 antibody on the support, it is preferred to use the anti-CD16 antibody solution in a volume in consideration of the surface area or the like of the solid-phase support. For example, approximately 15 mL of 1 µg/mL anti-CD16 antibody solution can be used for immobilization on a plastic flask having an inner wall surface area of 150 cm². Alternatively, 5 mL and 10 mL of the solution can be used for culture flasks having inner wall surface areas of 25 cm² and 75 cm², respectively. The anti-CD16 antibody is attached to the support by subsequent incubation at 37°C for 12 to 24 hours. Alternatively, a commercially available antibody immobilization kit or the like may be used. For example, CarboLink (Pierce Biotechnology, Inc.) can be used. Such an immobilization kit is useful for supports made of a material difficult to attach to antibodies.

After the immobilization of the anti-CD16 antibody on the support, desirably, the support with the anti-CD16 antibody immobilized thereon is washed, if necessary, to remove the anti-CD16 antibody solution. For example, the support can be washed several times, for example, approximately 2 to 5 times, with an appropriate amount of PBS. Such a culture container comprising the anti-CD16 antibody thus immobilized on the support can be stored at 0°C to 8°C, preferably 3°C to 6°C, and thereby used for approximately 1 month without reducing or inactivating the avidity of the antibody.

For stimulation with the anti-CD3 antibody, an OK432 solution can be added at, for example, 0.01 ng/mL to 1000 ng/mL, preferably 0.1 ng/mL to 10 ng/mL, more preferably 1 ng/mL, in terms of the final concentration to the culture solution containing PBMCs.

For stimulation with OK432, an OK432 solution can be added at, for example, 0.005 KE/mL to 0.02 KE/mL, preferably 0.008 KE/mL to 0.015 KE/mL, more preferably 0.01 KE/mL, in terms of the final concentration to the culture solution containing PBMCs. The OK432 solution can be prepared by dissolving Picibanil (5 KE/vial; Chugai Pharmaceutical Co., Ltd.) in 2 mL of water (e.g., injectable water).

For stimulation with the cytokine, one type of cytokine may be added thereto, or a combination of several types of cytokines may be added thereto. In consideration of cost, etc., it is preferred to add only IL-2. The amount of, for example, IL-2, added is preferably in the range of 100 units/mL to 2000 units/mL, in terms of the final concentration. This is because: an amount smaller than 100 units/mL is insufficient for inducing growth stimulation; and an amount larger than 2000 units/mL does not offer the growth of NK cells according to increase in IL-2 concentration. This amount is preferably in the range of 700 units/mL to 2000 units/mL.

When the NK cell growth-stimulating factors further comprise a bisphosphonate derivative or a salt thereof, or a hydrate thereof, 4 mg/vial of a zoledronic acid hydrate injection (2.94 µmol/mL; Novartis Pharma K.K.) can be used directly. Alternatively, zoledronic acid can be added to the medium at, for example, 1 µM/mL to 10 µM/mL, preferably 3 µM/mL to 7 µM/mL, more preferably 5 µM/mL, in terms of the final concentration.

For sufficiently stimulating PBMCs, it is preferred to keep the blood at a physiological cell temperature (usually, 37°C) for 1 to 3 days after the addition of each of these NK cell growth-stimulating factors. In this period, which may overlap with the period of the subsequent culture step, the NK cells, etc. can be cultured with stimulation applied thereto.

During the period for which the blood is kept at a physiological cell temperature, high-temperature stimulation may be applied thereto at 38°C to 40°C for a period of 10 hours to 30 hours. This high-temperature stimulation can further activate the NK cells. A temperature lower than 37°C at which the blood is kept in the stimulation step is not preferable because the temperature fails to sufficiently activate lymphocytes. A temperature higher than 40°C is not preferable because the temperature makes lymphocytes more likely to be degenerated or damaged by heat.

Means of keeping the blood at a predetermined temperature is not particularly limited as long as the blood can be kept at the constant temperature by this means. Examples thereof include means by which the blood together with its container is kept at a predetermined temperature using a CO₂ incubator.

### 1-2-2. Culture step

The "culture step" is the step of culturing the blood at a physiological cell temperature after the stimulation step. The feature of this step is that the number of the NK cells is increased with their growth/activation maintained.

The "physiological cell temperature" refers to the optimum temperature for culturing the blood. The physiological cell temperature is usually the body temperature of a mammal that has provided the blood used. Thus, when the mammal is a human, this temperature is generally 37°C and may be the temperature with a tolerance of less than ±0.5°C. This is because the internal temperature of a thermostat might fluctuate within this temperature range.

This step can be achieved by culturing the culture solution that has undergone the stimulation step, at a physiological cell temperature. At the initial stage of this step, the period for which the blood cells are sufficiently stimulated with the NK cell growth-stimulating factors added in the stimulation step can be secured and also serve as the period for which these cells are cultured. Then, it is preferred to temporarily remove the NK cell growth-stimulating factors from the medium to thereby cancel the stimulation step. This is because, although most of factors such as cytokines can continue to impart growth/activation-inducting stimulation to the NK cells even in the culture step, the long-term stimulation of the NK cells with the anti-CD16 antibody, OK432, the anti-CD3 antibody, and/or zoledronic acid or the like might have undesired influence, for example, cell death (e.g., apoptosis), on NK cell enrichment. These stimulating factors can be removed by a method involving, for example, temporarily collecting PBMCs from the culture solution after the stimulation step and then transferring these PBMCs to a new culture solution free from the anti-CD16 antibody, OK432, the anti-CD3 antibody, and optional zoledronic acid or the like. The removal of the factors is achieved by centrifuging the culture solution that has undergone the stimulation step and removing the supernatant. Its specific method can follow a medium replacement method described below.

The culture is performed for a period from 7 days to 21 days in a 5% CO₂ incubator that satisfies the physiological cell temperature condition. This is because: 6-day or shorter culture is insufficient for the growth of NK cells; and in 22-day or longer culture, the growth of NK cells reaches the resting phase or such long-term culture makes cells more likely to be damaged (e.g., deteriorated (aged)).

For the culture period, it is preferred to add a fresh medium or replace the medium by a fresh medium at regular intervals of 2 days to 5 days. As a specific example of the medium replacement, first, the culture solution containing PBMCs after the stimulation step is transferred to an already sterilized centrifuge tube. Subsequently, the tube is centrifuged at approximately 1200 rpm at room temperature for approximately 8 minutes, and the supernatant is then removed to collect PBMCs or NK cells as precipitates. The collected PBMCs or NK cells are transferred at a cell density of 0.6 to 1.0 ×10⁶ cells/mL to a fresh culture solution containing IL-2 and plasma. In this context, the cytokine such as IL-2 can be added thereto at approximately 300 units/mL to approximately 700 units/mL in terms of the final concentration. This is because the NK cells have already been activated after the stimulation step and produce cytokines such as IL-2 in themselves.

The medium used in the culture can be any general medium for use in cell culture as a rule. OpTmizer medium (Invitrogen Corp.) is preferable.

After the culture, the culture solution is confirmed to be free from contamination with bacteria or endotoxin. The presence or absence of bacteria can be examined by colony formation assay, while the presence or absence of endotoxin can be examined by colorimetry such as commercially available ELISA or by a suspension method such as limulus test.

### 1-3. Effect

The method for producing an NK cell-enriched blood preparation according to this embodiment can produce an NK cell-enriched blood preparation from blood collected from an organism. Particularly, the production method can provide an NK cell-enriched blood preparation that has 3 or more times higher than the growth rate and activity of NK cells obtained in the method for producing an NK cell-enriched blood preparation according to the prior application.

According to the method for producing an NK cell-enriched blood preparation according to this embodiment, the essential step of keeping blood at a predetermined temperature for a predetermined time (activation step; which corresponds to the optional high-temperature stimulation in the stimulation step of the present invention) in the prior application is no longer essential. As a result, an incubator set to a predetermined temperature necessary for the activation step is not necessarily required. This can drastically reduce burdens from an equipment standpoint in research facilities where the present invention is carried out, or burdens in terms of operation/management by an operator.

According to the production method using a bisphosphonate derivative as one NK cell growth-stimulating factor, γδT cells can also be grown at the same time with NK cells, as shown in Example 3 described later. The γδT cells are also effective for cellular immunotherapy, as in NK cells. Thus, the production method can produce synergistic effect, i.e., the growth of NK cells and γδT cells, which has not been achieved by the production of the prior application.

Moreover, the production method can minimize physical burdens on donors because the blood collected from an organism may be peripheral blood.

The production method does not require particular special equipment or the like and can utilize regular equipment or the like installed in general testing facilities, research facilities, etc., for cell culture. In addition, any of necessary reagents, etc. can be obtained easily. Accordingly, the production method of this embodiment can be carried out advantageously in research facilities capable of aseptic manipulation, such as clean room, substantially without the need of initial equipment investment or the like.

The NK cell-enriched blood preparation obtained by the production method of this embodiment is capable of preventing the recurrence of cancer or effectively treating advanced cancer in actual clinical experiments. In addition, the blood preparation that can be provided is safe in such a way that the administration of the blood preparation has been confirmed to have no adverse reaction.

### 2. NK cell-enriched blood preparation

### 2-1. Summary

The second embodiment of the present invention relates to an NK cell-enriched blood preparation obtained by the production method of the first embodiment.

### 2-2. Constitution

The NK cell-enriched blood preparation of this embodiment can be obtained from the culture solution that has undergone the culture step in the first embodiment. However, the NK cell-enriched blood preparation does not require the medium used in the culture or the growth-stimulating factors added to the medium. Thus, for use of the NK cell-enriched blood preparation, it is preferred to remove the medium and the growth-stimulating factors as much as possible from the culture solution to obtain grown/activated NK cells, etc. As a specific example, the medium and the growth-stimulating factors are removed by a method involving first transferring the culture solution containing the grown/activated NK cells to an already sterilized centrifuge tube, which is then centrifuged at 1200 rpm at room temperature for approximately 8 minutes to remove the medium in a supernatant containing the growth-stimulating factors. The NK cells can be collected as precipitates. It is preferred to wash the collected NK cells two or more times with PBS (-). The number of the NK cells thus washed is counted using a hemacytometer and adjusted with 10 mL to 200 mL of a lactate Ringer solution or saline. In this way, the NK cell-enriched blood preparation of this embodiment can be obtained. If necessary, cytokines or the like may be added to the blood preparation.

For obtaining sufficient effects using the blood preparation of this embodiment, it is preferred that 70% or more of the number of the NK cells contained therein should be in an activated state. The activation of the NK cells can be determined by examining cytotoxic activity or activation marker expression using a leukemia cell line K562. A marker known in the art, such as CD69, can be used as the activation marker. An antibody against each marker can be used in the detection thereof.

### 2-3. Effect

According to the NK cell-enriched blood preparation of this embodiment, an NK cell-enriched blood preparation containing activated NK cells as many as 10 × 10⁷ to 100 × 10⁹ cells can be obtained from 20 mL to 60 mL of peripheral whole blood.

In the NK cell-enriched blood preparation of this embodiment, approximately 90% of the NK cells are active. This corresponds to 10 or more times that in the blood of a healthy adult.

The NK cell-enriched blood preparation of this embodiment may be used immediately after its production or may be stored either for a predetermined period at a temperature of 0°C to 8°C or for a period as long as several years at a ultralow temperature (approximately -80°C) or in liquid nitrogen after being supplemented with a storage solution or the like. A commercially available lymphocyte storage solution can be used conveniently as the storage solution. For example, Bambanker (Nippon Genetics Co., Ltd.) or KM Banker II (Cosmo Bio Co., Ltd.) can be used.

### 3. Composition for NK cell enrichment

### 3-1. Summary

The third embodiment of the present invention relates to a composition for NK cell enrichment. The feature of this embodiment is that the composition comprises NK cell growth-stimulating factors comprising an anti-CD16 antibody, OK432, an anti-CD3 antibody, and a cytokine. Also, the composition for NK cell enrichment of this embodiment may further comprise a bisphosphonate derivative or a salt thereof, or a hydrate thereof, particularly, zoledronic acid. The composition for NK cell enrichment of this embodiment can be added to blood, preferably a medium containing PBMCs, to thereby conveniently and efficiently growing/activating NK cells in the medium.

### 3-2. Constitution

The "composition for NK cell enrichment" refers to a composition capable of growing/activating NK cells present in a medium through its addition to the medium.

The composition for NK cell enrichment of this embodiment comprises the anti-CD16 antibody, the OK432, the anti-CD3 antibody, and the cytokine described in the first embodiment and optionally comprises the bisphosphonate derivative represented by the formula 1 or the salt thereof, or the hydrate thereof. The anti-CD3 antibody is preferably an anti-CD3 monoclonal antibody such as muromonab-CD3. The cytokine is preferably a compound selected from the group consisting of IL-2, IL-12, IL-15, and IL-18, more preferably IL-2. The bisphosphonate derivative is preferably a compound selected from the group consisting of zoledronic acid, pamidronic acid, alendronic acid, risedronic acid, ibandronic acid, incadronic acid, and etidronic acid, more preferably, zoledronic acid. The composition for NK cell enrichment may additionally incorporate medium components for lymphocytes, such as RPMI-1640, a pH stabilizer, an antibiotic, etc.

These components for the composition can be mixed in amounts that give their respective predetermined final concentrations when added to a predetermined amount of a medium. Specifically, these components can be mixed so that: the anti-CD16 antibody gives a final concentration of 0.01 µg/mL to 100 µg/mL, preferably 0.1 µg/mL to 10 µg/mL, more preferably 1 µg/mL; OK432 gives a final concentration of 0.005 KE/mL to 0.02 KE/mL, preferably 0.008 KE/mL to 0.015 KE/mL, more preferably 0.01 KE/mL; the anti-CD3 antibody (preferably, muromonab-CD3) gives a final concentration of 0.01 ng/mL to 1000 ng/mL, preferably 0.1 ng/mL to 10 ng/mL, more preferably 1 ng/mL; and the cytokine (preferably, IL-2) gives a final concentration of 500 U/mL to 2000 U/mL, preferably 700 U/mL to 1500 U/mL, more preferably 1000 U/mL. When the composition further comprises a bisphosphonate derivative or a salt thereof, or a hydrate thereof (preferably, zoledronic acid), this component can be mixed therewith at a final concentration of 1 µM/mL to 10 µM/mL, preferably 3 µM/mL to 7 µM/mL, more preferably 5 µM/mL.

The dosage form of the composition is not particularly limited. The composition can be in a liquid form dissolved in an appropriate buffer, in a powdery form, or in the form of tablets prepared from a powder supplemented with an appropriate excipient, etc. Alternatively, the composition may be a mixture of different forms. For example, the composition is in a dosage form in which the anti-CD16 antibody immobilized on a solid-phase support such as plastic beads is mixed with a solution containing the OK432, the anti-CD3 antibody, and the cytokine, and optionally, the bisphosphonate derivative represented by the formula 1 or the salt thereof, or the hydrate thereof.

### 3-3. Effect

According to the composition for NK cell enrichment of this embodiment, NK cells can be activated and grown by simple procedures of addition to a predetermined amount of an appropriate cell culture solution containing the NK cells and subsequent culture.

### 4. Kit for production of NK cell-enriched blood

### 4-1. Summary

The fourth embodiment of the present invention relates to a kit for production of NK cell-enriched blood. The kit of this embodiment can be used in the culture of blood, preferably PBMCs, to thereby conveniently and easily produce an NK cell-enriched blood preparation.

### 4-2. Constitution

The kit for production of NK cell-enriched blood of this embodiment comprises the anti-CD16 antibody, the OK432, the anti-CD3 antibody, and the cytokine described in the first embodiment and optionally comprises the bisphosphonate derivative represented by the formula 1 or the salt thereof, or the hydrate thereof. The kit for production of NK cell-enriched blood may additionally incorporate sterile water or a buffer for dissolving each NK cell growth-stimulating factor, an instruction manual, etc.

The anti-CD16 antibody and the anti-CD3 antibody incorporated in this kit are not particularly limited as long as the antibodies specifically recognize CD16 and CD3, respectively, and bind thereto. These antibodies may be any of monoclonal and polyclonal antibodies. A monoclonal antibody is preferable. It is preferred that the anti-CD16 antibody should be immobilized on an appropriate solid-phase support. Specific examples of the anti-CD3 antibody incorporated in this kit include muromonab-CD3 (trade name: Orthoclone OKT3 (registered trademark), Janssen Pharmaceutical K.K.). The cytokine is preferably a compound selected from the group consisting of IL-2, IL-12, IL-15, and IL-18, more preferably IL-2. The bisphosphonate derivative is preferably a compound selected from the group consisting of zoledronic acid, pamidronic acid, alendronic acid, risedronic acid, ibandronic acid, incadronic acid, and etidronic acid, more preferably zoledronic acid.

These NK cell growth-stimulating factors can be incorporated alone or in combination of two or more thereof in the kit. For example, the NK cell growth-stimulating factors other than the anti-CD 16 antibody may be incorporated in one portion in the kit. The state of each NK cell growth-stimulating factor is not particularly limited. One NK cell growth-stimulating factor may be in a liquid state while the other NK cell growth-stimulating factors may be in a solid state. Particularly, it is preferred that the anti-CD16 antibody should be incorporated therein in a form immobilized on an appropriate solid-phase support such as plastic beads.

### 5. Cellular immunotherapy to treat disease

### 5-1. Summary

The fifth embodiment of the present invention relates to cellular immunotherapy for treating a disease, involving administering the NK cell-enriched blood preparation produced in the first embodiment to an organism to enhance its immunity.

### 5-2. Constitution

This embodiment relates to cellular immunotherapy involving administering the NK cell-enriched blood preparation obtained by the production method of the first embodiment to an organism.

The "cellular immunotherapy" according to this embodiment refers to a method for treating a disease, involving administering the NK cell-enriched blood preparation obtained by the production method of the first embodiment to an organism to enhance the immunity of the organism. Particularly, for the cellular immunotherapy of this embodiment, it is preferred to be predicated on adoptive immunotherapy. This is because the adoptive immunotherapy is substantially free from the risk of rejection, as described above.

The NK cell-enriched blood preparation to be administered contains a larger number of lymphocytes having immunity against cancer, viral infection, or fungal infection than the average number thereof in usual blood per unit volume. In this context, the "cancer" means general malignant tumor. The cancer corresponds to, for example, epithelial tumor, sarcoma, leukemia, and myeloma. The "viral infection" refers to general disease caused by infection with a virus and particularly corresponds to the intractable chronic viral infection and acute viral infection. Examples of the intractable chronic viral infection include HIV infection causative of AIDS, chronic viral hepatitis, and human papillomavirus infection causative of uterine cervix cancer. Examples of the acute viral infection include viral respiratory infection such as influenza, and acute viral infection in an immunodeficient state. The "fungal infection" refers to infection with filamentous bacteria, yeast, or the like. Examples thereof include candidal infection, blastomycosis, and histoplasmosis.

The "lymphocyte having immunity" means a lymphocyte having fortified functions in the immune system. Such lymphocytes correspond to, for example, NK cells, killer T cells, γδT cells, and NKT cells that have been activated to be cytotoxic. In this context, the "average value in usual blood per unit volume" means the average number per unit volume of blood cells having immunity against cancer, viral infection, or fungal infection generally observed in the blood of a healthy individual. For example, approximately 5 × 10⁵ NK cells on average are present per mL of blood of a healthy adult individual.

### 5-3. Method

Hereinafter, a method for administering the NK cell-enriched blood preparation in the cellular immunotherapy of this embodiment will be described by taking adoptive immunotherapy as an example. The administration method is basically the same as a known method performed in the conventional adoptive immunotherapy except that the NK cell-enriched blood preparation of the second embodiment is administered. Thus, the administration method can be performed according to that of the adoptive immunotherapy known in the art. Examples thereof include methods involving administering the blood preparation produced by the method for producing an NK cell-enriched blood preparation according to the second embodiment from blood collected from a patient, into the body of the patient using, for example, intravenous injection or drip infusion approximately 2 weeks later.

One dose of the NK cell-enriched blood preparation according to this embodiment can be a volume containing NK cells in the range of 20 × 10⁷ to 5 × 10⁹ cells for a human. This dose is intended for a general adult. For actual administration, it is preferred to appropriately adjust the dose in consideration of the age, sex, body weight, disease conditions, body strength, etc., of a recipient of the blood preparation.

One example of the cellular immunotherapy of this embodiment includes 1 course (6 cycles) or longer of continuous administration at approximately 2-week intervals with the above-described administration method defined as one cycle. Cellular immunotherapy other than adoptive immunotherapy can also be performed in the same way as above except that an NK cell-enriched blood preparation obtained from a non-self organism is administered.

### 5-4. Effect

The cellular immunotherapy of this embodiment has high efficacy on the healing of a disease such as cancer, compared with many conventional immunotherapy methods, particularly, adoptive immunotherapy. A person skilled in the conventional adoptive immunotherapy can carry out the cellular immunotherapy of this embodiment without acquiring particular skills because this cellular immunotherapy can be operated by the same basic technique, etc., as in the conventional adoptive immunotherapy.

### <Examples>

Hereinafter, the present invention will be described specifically with reference to Examples. Examples below are provided merely for illustrative purposes of the present invention, and the present invention is not intended to be limited to these Examples by any means. In this context, small experimental errors and deviations are tolerated for numeric values as to temperature, amount, time, etc., used in these Examples.

### [Example 1]

### <Method for producing NK cell-enriched blood preparation>

The first embodiment of the present invention will be described with reference to a specific example of the method for producing the blood preparation used in adoptive immunotherapy. In Examples 1 to 4 of the present specification, healthy individuals were used as donors instead of actual individuals to be treated such as cancer patients.

### (1) Preparation of autologous plasma

First, autologous plasma for culture was prepared. 40 mL of peripheral whole blood was collected from the vein of each donor into a blood collection tube supplemented with 50 units/mL heparin. The collected peripheral whole blood was transferred to a sterile conical centrifuge tube and centrifuged at 3000 rpm for 10 minutes. Then, the supernatant was separated as plasma. To the remaining blood cell components after the plasma collection, sterile PBS (-) or a medium for culture was added in an amount 3 times that of the whole blood before plasma separation to prepare a "blood cell component solution", which was in turn used in the subsequent preparation of peripheral blood mononuclear cells. The plasma was inactivated by treatment at 56°C for 30 minutes and further centrifuged at 3000 rpm for 10 minutes to remove platelet, etc. Then, the plasma was stored at 4°C. This plasma was intended for addition to a cell culture medium and used in a necessary amount every time a medium was prepared.

### (2) Preparation of peripheral blood mononuclear cells (PBMCs)

A specific gravity solution was layered onto the blood cell component solution. Erythrocytes or granulocytes were removed using a density-gradient centrifugation method to isolate PBMCs. The specific gravity solution used was Ficoll-Paque PLUS (GE Healthcare (formerly Amersham Biosciences Corp.)), and the operational procedures followed the protocol supplied with the kit. The collected PBMCs were washed 2 or 3 times by the addition of 30 mL of serum-free PBS (-) or a medium for cultured cells. The medium for cultured cells used was, for example, a serum-free RPMI1640 medium. After the washing, an aliquot was sampled from the obtained suspension of PBMCs and stained with a Turk's solution, and the number thereof was then counted using a hemacytometer. As a result, 3.4 × 10⁷ PBMCs were collected from 40 mL of peripheral whole blood. The peripheral blood mononuclear cells thus collected were added and suspended at a cell density of 1 × 10⁶ cells/mL to OpTmizer (Life Technologies Corp.) medium supplemented with 5% (V/V) of the autologous plasma.

### (3) Stimulation step

0.2 mg of an anti-CD16 antibody (Clone 3 GB, Beckman Coulter, Inc.) was dissolved in 1 mL of sterile distilled water. Since this anti-CD16 antibody is not a sterile product, this solution was sterilized by filtration through a 0.22 µm filter. The solution was adjusted to 1 µg/mL in terms of the final concentration by the addition of 199 mL of sterile distilled water, and then mixed. After filtration, 5 mL of the anti-CD 16 antibody solution was placed in a 25 cm² culture flask and left standing overnight at 37°C to immobilize the anti-CD16 antibody in this solution onto the inner wall of the flask. Then, the solution was discarded, and the inside of the flask was washed twice with sterile PBS (-).

11.2 mL of the prepared suspension of PBMCs was transferred into the flask. Subsequently, 11.2 µL of a 1000-fold diluted solution of an anti-CD3 antibody (Orthoclone OKT3, Janssen Pharmaceutical K.K.), 44.8 µL of 4 µL/mL (final concentration) aqueous OK432 (Picibanil; Chugai Pharmaceutical Co., Ltd.) solution, and 8.7 µL of 900 units/µL IL-2 (Proleukin; Chiron Corp.) solution were added to the suspension of PBMCs, and the mixture was sufficiently stirred.

In order to sufficiently stimulate PBMCs with each of the NK cell growth-stimulating factors, the culture flask was transferred to a 5% CO₂ incubator preset to a chamber temperature of 37°C, and kept for 3 days.

### (4) Culture step

In order to remove the NK cell growth-stimulating factors from the culture solution, 11.2 mL of the culture solution was then collected into a conical centrifuge tube and centrifuged at 1200 rpm for 8 minutes. After the centrifugation, the medium in the supernatant was removed, and the cell pellet was suspended in 8.2 mL of OpTmizer medium containing 5% (V/V) autologous plasma containing 700 units/µL IL-2. The collected cell suspension was transferred to a new anti-CD16 antibody-unimmobilized flask and then cultured again for 14 days in a 5% CO₂ incubator set to 37°C. The OpTmizer medium containing 5% (V/V) autologous plasma was replaced by a fresh one every 2 to 4 days. In this way, the NK cell-enriched blood preparation of the second embodiment of the present invention was prepared.

For actually using the NK cell-enriched blood preparation, it is required to perform a contamination test or pretreatment. Hereinafter, their procedures will be described simply.

### (Contamination test)

The presence or absence of endotoxin in the culture solution was confirmed using Limulus ES-II (Wako Pure Chemical Industries, Ltd.) according to the protocol supplied with the kit. Also, the presence or absence of bacteria or mold was confirmed by colony formation assay using an aliquot of the culture solution applied to an agar medium.

### (Pretreatment of NK cell-enriched blood preparation)

Two weeks after culture, the culture solution was transferred to a centrifuge tube and centrifuged at 1200 rpm for 10 minutes, and the supernatant was then discarded. The precipitates were suspended by the addition of 50 mL of PBS (-) and centrifuged again at 1200 rpm for 10 minutes, and the supernatant was then discarded. This operation was performed 3 repetitive times to remove the medium components. Finally, the residue was suspended in 70 mL of lactate Ringer solution. In this way, the NK cell-enriched blood preparation was obtained as the final product. The blood preparation had an NK cell growth rate of approximately 500 times after 14 days.

### [Example 2]

### <Growth ofNK cell - (1) ->

In order to confirm that the method for producing an NK cell-enriched blood preparation according to the present invention did not require high-temperature stimulation, the growth rate ofNK cells was examined.

### (Method)

In this Example, two samples shown below were examine for the growth rate of NK cells in blood obtained from each of two donors (#1 and #2) to compare the method of the present invention using an anti-CD3 antibody (Orthoclone OKT3, Janssen Pharmaceutical K.K.) solution with the method of the prior application comprising an activation step involving incubation at 39°C without using the anti-CD3 antibody.

Sample a: The NK cell growth-stimulating factors used were 1 µg/mL anti-CD16 antibody, 0.01 KE/mL OK432, and 700 units/mL IL-2 (concentrations were all indicated by the final concentrations). The NK cell growth-stimulating factors in this sample correspond to growth-stimulating factors used in the prior application. This approach involves high-temperature stimulation in which the cells are kept at 39°C at the start of culture for 16 hours.

Sample b: The NK cell growth-stimulating factors used were 1 µg/mL anti-CD16 antibody, 0.01 KE/mL OK432, a 1 ng/mL solution of an anti-CD3 antibody (muromonab-CD3: trade name: Orthoclone OKT3 (registered trademark), Janssen Pharmaceutical K.K.), and 700 units/mL IL-2 (concentrations were all indicated by the final concentrations). The NK cell growth-stimulating factors in this sample correspond to the NK cell growth-stimulating factors of the present invention. Unlike the production method according to the prior application, this approach does not involve high-temperature stimulation at 39°C.

The basic operation of the method for producing an NK cell-enriched blood preparation was the same as in Example 1 except for difference in the composition of each sample described above and steps. The day when PBMCs were suspended at a cell density of 1 × 10⁶ cells/mL in OpTmizer medium was defined as day 0. The day when stimulation and culture were initiated by stimulation with each stimulating factor and addition of 10% autologous plasma to the medium was defined as day 0. At culture (including high-temperature stimulation) days 0, 3, 5, 7, 9, 12, and 14, an aliquot of each culture solution was collected, and the total number of cells in the culture solution was determined.

NK cells in each culture solution were assayed using a flow cytometry analysis method basically at day 0 and day 14 and at day 8 and day 21 for some samples. Specifically, the NK cells in the blood preparation were immunostained using a combination of fluorescent material-labeled monoclonal antibodies (PC5- or ECD-labeled anti-CD3 antibody and PE- or PC5-labeled anti-CD56 antibody; Immunotech). The immunostaining was performed by adding, to the cell suspension, each antibody in an amount recommended by the document supplied with the antibody, and staining the cells at room temperature for 15 minutes in the dark, followed by centrifugation and washing off of the supernatant containing the fluorescently labeled antibodies. Subsequently, the kinetics of the NK cells were assayed by flow cytometry using Cytomics FC500 (Beckman Coulter, Inc.) based on the combination of the antibodies. The assay data was analyzed by CXP analysis.

### (Results)

The results are shown in Figures 1, 2, and 3 and Table 1.

**[Table 1]**

| At start of culture (day 0) | | | | | | |
|---|---|---|---|---|---|---|
| Donor | Sample | PBMCs (× 10⁶ cells) | NK % | Absolute number of NK cell (× 10⁶ cells) | | |
| #1 | a | 12.9 | 8.7 | 1.1 | | |
| | b | | | | | |
| #2 | a | 11.2 | 23.5 | 2.6 | | |
| | b | | | | | |
| | | | | | | |

| At culture day 14 | | | | | | |
|---|---|---|---|---|---|---|
| Donor | Sample | The number of cell (× 10⁶ cells) | NK % | Absolute number of NK cell (× 10⁶ cells) | NK cell growth rate (-fold) | Cytotoxic activity (E/T=3:1) |
| #1 | a | 1800 | 68.3 | 1229.4 | 1117.6 | 95 |
| | b | 15526.4 | 24.8 | 3850.5 | 3500.5 | 87 |
| #2 | a | 152.5 | 93.8 | 143 | 55 | 87 |
| | b | 2188.8 | 58.9 | 1289.2 | 495.8 | 87 |

As shown in Figure 2, both donors #1 and #2 started to differ in the number of cells between samples a and b from around day 5 after the start of culture. NK cells in sample b cultured with the NK cell growth-stimulating factors comprising the anti-CD3 antibody Orthoclone OKT3 exhibited significant increase from day 7 after the start of culture and reached, as shown in Table 1, approximately 9 times (donor #2) and 3.1 times (donor #1) the absolute number of NK cells in sample a at day 14. These results demonstrated that the production method of the present invention can more efficiently grow NK cells than the conventional method without requiring high-temperature stimulation at 39°C.

### [Example 3]

### <Growth ofNK cell - (2) ->

The NK cell-enriched blood preparation of the present invention was examined for effects brought about by the further addition of a bisphosphonate derivative as an NK cell growth-stimulating factor and for effects brought about by long-term culture.

### (Method)

Two samples shown below were examined for the growth rate of NK cells.

Sample b: The NK cell growth-stimulating factors used were 1 µg/mL anti-CD16 antibody, a 1 ng/mL solution of an anti-CD3 antibody, 0.01 KE/mL OK432, and 700 units/mL IL-2 (concentrations were all indicated by the final concentrations). In other words, this sample was treated in the same way as in sample b of Example 2.

Sample c: The NK cell growth-stimulating factors used were 1 µg/mL anti-CD16 antibody, a 1 ng/mL solution of an anti-CD3 antibody, 0.01 KE/mL OK432, 5 µM/mL bisphosphonate derivative (zoledronic acid; trade name: Zometa (registered trademark), Novartis Pharma K.K.), and 700 units/mL IL-2 (concentrations were all indicated by the final concentrations). Sample c is sample b further supplemented with the bisphosphonate derivative.

The number of NK cells was determined using a flow cytometry analysis method as in Example 2.

Also, the number of γδT cells was determined using a flow cytometry analysis method as in the NK cells of Example 2. The γδT cells in the blood preparation were immunostained using a combination of fluorescent material-labeled monoclonal antibodies (FITC-labeled anti-Vy9 antibody and PC5- or ECD-labeled anti-CD3 antibody; Immunotech). The immunostaining was performed by adding, to the cell suspension, each antibody in an amount recommended by the document supplied with the antibody, and staining the cells at room temperature for 15 minutes in the dark, followed by centrifugation and washing off of the supernatant containing the fluorescently labeled antibodies. Subsequently, the kinetics of the γδT cells were assayed by flow cytometry using Cytomics FC500 (Beckman Coulter, Inc.) based on the combination of the antibodies. The assay data was analyzed by CXP analysis.

### (Results)

The results are shown in Figures 4, 5, 6, 7, 8, and 9 and Table 2.

**[Table 2]**

| At start of culture (day 0) | | | | | | | | | |
|---|---|---|---|---|---|---|---|---|---|
| Donor | Sample | PBMCs (× 10⁶ cells) | NK % | Absolute number of NK cell (× 10⁶ cells) | γδT % | Absolute number of γδT cell (× 10⁶ cells) | | | |
| #1 | b | 12.9 | 8.7 | 1.1 | 1 | 1.3 | | | |
| | c | | | | | | | | |
| #2 | b | 11.2 | 23.5 | 2.6 | 1.5 | 0.17 | | | |
| | c | | | | | | | | |

| At culture day 14 | | | | | | | | | |
|---|---|---|---|---|---|---|---|---|---|
| Donor | Sample | The number of cell (× 10⁶ cells) | NK % | Absolute number of NK cell (× 10⁶ cells) | NK cell growth rate (-fold) | γδT % | Absolute number of γδT cell (× 10⁶ cells) | γδT cell growth rate (-fold) | Cytotoxic activity (E/T=3:1) |
| #1 | b | 15526.4 | 24.8 | 3850.5 | 3500.5 | 2.1 | 326 | 250.8 | 87 |
| | c | 13094.4 | 23.5 | 3077.2 | 2797.5 | 5.4 | 707.1 | 543.9 | 82 |
| #2 | b | 2188.8 | 58.9 | 1289.2 | 495.8 | 1.2 | 26.3 | 154.7 | 87 |
| | c | 2144 | 56.4 | 1209.2 | 465 | 9.2 | 197.2 | 1160 | 89 |

| At culture day 21 | | | | | | | | | |
|---|---|---|---|---|---|---|---|---|---|
| Donor | Sample | The number of cell (× 10⁶ cells) | NK % | Absolute number of NK cell (× 10⁶ cells) | NK cell growth rate (-fold) | γδT % | Absolute number of γδT cell (× 10⁶ cells) | γδT cell growth rate (-fold) | Cytotoxic activity (E/T=3:1) |
| #1 | b | 64305 | 27.9 | 17941.1 | 16310 | 1.5 | 964.5 | 741.9 | 86 |
| | c | 61440 | 35.3 | 21688.3 | 19716.6 | 3.4 | 2089 | 1606.9 | 86 |

As shown in Table 2, no significant difference in the growth rate of NK cells was observed between samples b and c at both culture day 14 and day 21. Both the samples exhibited continuous growth until culture day 14 to day 21. On the other hand, the growth rate of γδT cells was shown to be higher in sample c supplemented with zoledronic acid as a bisphosphonate derivative than nonsupplemented sample b at both culture day 14 and day 21.

The results described above demonstrated that the method for producing an NK cell-enriched blood preparation, comprising adding the bisphosphonate derivative can produce a γδT cell growth rate approximately 2.2 to 7.5 times higher than that of the production method that does not involve such addition during culture for 14 days, even if these methods are performed under the same conditions.

In addition, this method was confirmed to yield cultured cells without qualitative deterioration even after a culture period as long as 21 days. These results demonstrated that a culture period can be extended in order to obtain a larger number of NK cells and γδT cells and that the possible maximum culture period is at least 21 days.

### [Example 4]

### <Assay of activated NK cell>

The activation of NK cells in the NK cell-enriched blood preparation of the present invention was assayed on the basis of cytotoxic activity against a K562 cell line, which was targeted by NK cells.

### (Method)

First, established leukemia cell line K562 cells were labeled with a fluorescent dye Calcein-AM. The labeling was performed by incubation at 37°C for 30 minutes in a RPMI-1640 medium (containing 10% fetal bovine serum) supplemented with a 1/100 volume of Calcein-AM solution (Dojindo Laboratories). The cells thus labeled were washed with PBS (-) and used as target cells. Next, NK cells in the sample a-, b-, and c-derived NK cell-enriched blood preparations produced by the methods of Examples 2 and 3 were separately used as effector cells (E). These effector cells were adjusted to their respective predetermined values in terms of the ratio (E/T ratio) to the target K562 cells (target cells: T), then separately placed in a 96-well plate, and reacted at 37°C for 2 hours at a CO₂ concentration of 5%. After the reaction, the amounts of the target cells that retained fluorescence, i.e., survived, were detected on the basis of their fluorescence intensities using Terascan VP (Minerva Tech K.K.). The value of cytotoxic activity against K562 was calculated by comparison with a control before cytotoxicity, i.e., fluorescence intensity from a state nonsupplemented with effector cells.

### (Results)

The cytotoxic activity of the NK cells produced by the methods of Examples 2 and 3 is shown in Tables 1 and 2. The cytotoxic activity of the NK cells in samples a, b, and c produced from donor #2 is shown in Figure 10. The cytotoxic activity at day 14 and day 21 of the NK cells produced by the method of Example 3 from donor #1 is shown in Figure 11. The E/T ratios used are shown in the corresponding tables or diagrams.

As is evident from Tables 1 and 2 and Figures 10 and 11, the NK cells obtained in the method for producing an NK cell-enriched blood preparation according to the present invention had cytotoxic activity substantially equivalent to that of the method for producing an NK cell-enriched blood preparation according to the prior application. In addition, the cytotoxic activity of the NK cells was not reduced even after culture until day 21. These results demonstrated that the method for producing an NK cell-enriched blood preparation according to the present invention can provide the cytotoxic activity ofNK cells as high as that obtained by the production method according to the prior application, and can also yield a larger number of activated NK cells.

### [Example 5]

### <NK cell-enriched blood preparation prepared using cancer patient-derived blood and growth rates ofNK cell and γδT cell>

In this Example, an NK cell-enriched blood preparation was produced by the production method described in the first embodiment of the present invention using a cancer patient, which is an actual individual to be treated, as a donor, and examined for the growth rates of its NK cells and γδT cells.

The blood donors were seven 53- to 79-year-old male and female cancer patients having colon cancer, uterine body cancer, ovary cancer, or lung cancer at stages IIIc to IV. For specific information about each donor, see Table 3 described later. Needless to say, this experiment was conducted after obtainment of informed consent from each patient.

### (Method)

Two samples shown below were evaluated for the growth rates of NK cells and γδT cells.

Sample a: This sample was treated in the same way as in sample a of Example 2. Specifically, NK cell growth-stimulating factors used in this sample correspond to growth-stimulating factors used in the prior application. This approach involves high-temperature stimulation in which the cells are kept at 39°C at the start of culture for 16 hours.

Sample c: This sample was treated in the same way as in sample c of Example 3. Specifically, this sample is sample a further supplemented with a 1 ng/mL solution of an anti-CD3 antibody and 5 µM/mL bisphosphonate derivative (zoledronic acid; trade name: Zometa (registered trademark), Novartis Pharma K.K.) (concentrations were all indicated by the final concentrations). Unlike the production method according to the prior application, this approach does not involve high-temperature stimulation at 39°C.

The basic operation of the method for producing an NK cell-enriched blood preparation was the same as in Examples 1 to 3 except for cancer patients used as donors and the number of culture days. Thus, the specific description thereof is omitted here. The number of culture days was set to the range of 13 days to 22 days for each donor.

The numbers of NK cells and γδT cells in the culture solution were determined by a flow cytometry analysis method as in Example 2. Since specific procedures are the same as in Example 2, the description thereof is omitted here.

### (Results)

The results are shown in Table 3.

**[Table 3]**

| At start of culture (day 0) | | | | | | | | | | |
|---|---|---|---|---|---|---|---|---|---|---|
| Donor | Disease name | Age | Sex | Stage | Sample | PBMCs (× 10⁶ cells) | NK % | Absolute number of NK cell (× 10⁶ cells) | γδT % | Absolute number of γδT cell (× 10⁶ cells) |
| #3 | Colon cancer | 79 | Female | IV | a | 9.4 | 19.9 | 1.3 | 0.2 | 0.01 |
| | | | | | c | | | | | |
| #4 | Colon cancer | 79 | Female | IV | a | 9.4 | 19.9 | 1.3 | 0.2 | 0.01 |
| | | | | | c | | | | | |
| #5 | Uterine body cancer | 62 | Female | IV | a | 8.2 | 7 | 0.5 | 0.4 | 0.03 |
| | | | | | c | | | | | |
| #6 | Peritoneally disseminated ovary cancer | 58 | Female | IIIc | a | 14.2 | 8 | 47.6 | 1.6 | 1.2 |
| | | | | | c | | | | | |
| #7 | Lung cancer | 68 | Male | IV | a | 8.9 | 3.9 | 0.3 | 0.9 | 0.07 |
| | | | | | c | | | | | |
| #8 | Lung cancer metastasized to bone | 53 | Female | IV | a | 11 | 8.3 | 0.8 | 0.4 | 0.04 |
| | | | | | c | | | | | |
| #9 | Lung cancer | 64 | Male | IV | a | 11.4 | 14 | 1.2 | 0.1 | 0.01 |
| | | | | | c | | | | | |

| At completion of culture | | | | | | | | | | |
|---|---|---|---|---|---|---|---|---|---|---|
| Donor | Sample | The number of culture days | The number of cell (× 10⁶ cells) | NK % | Absolute number of NK cell (× 10⁶ cells) | NK cell growth rate (-fold) | γδT % | Absolute number of γδT cell (× 10⁶ cells) | γδT cell growth rate (-fold) | |
| #3 | a | 22 | 1224 | 97 | 1187.3 | 935 | 0.1 | 0.6 | 48 | |
| | c | | 2416 | 81.6 | 1971.5 | 1552 | 0.1 | 1.2 | 95 | |
| #4 | a | 22 | 1224 | 97 | 1187.3 | 934.8 | 0.1 | 1.2 | 95.9 | |
| | c | | 2416 | 81.6 | 1971.5 | 1552.2 | 0.1 | 2.4 | 189.3 | |
| #5 | a | 14 | 560 | 74.8 | 418.9 | 791.5 | 1.2 | 6.7 | 222.2 | |
| | c | | 2880 | 22.8 | 656.6 | 1240.8 | 6.2 | 178.6 | 5904.4 | |
| #6 | a | 21 | 98 | 48.6 | 47.6 | 49.4 | 1.2 | 1.2 | 6.1 | |
| | c | | 3040 | 43.1 | 1310.2 | 1358.5 | 1.8 | 54.7 | 283.7 | |
| #7 | a | 13 | 169 | 84.2 | 142.3 | 478.9 | 1 | 1.7 | 24.6 | |
| | c | | 1040 | 67 | 696.8 | 2345.2 | 14.8 | 153.9 | 2244.9 | |
| #8 | a | 17 | 360.2 | 91.1 | 328.1 | 401.1 | 3.4 | 12.2 | 310.6 | |
| | c | | 1930 | 56.3 | 1086.6 | 1328.3 | 4.5 | 86.9 | 2203 | |
| #9 | a | 18 | 640 | 40.2 | 257.3 | 214.1 | 0.4 | 2.6 | 298.2 | |
| | c | | 3424 | 42.7 | 1462 | 1216.6 | 0.1 | 3.4 | 398.9 | |

As shown in Table 3, sample c of the present invention exhibited higher growth rates of both NK cells and γδT cells than those of sample a in all the donors, though the values differed among individuals.

The results described above demonstrated that the method for producing an NK cell-enriched blood preparation according to the present invention can more efficiently grow NK cells and γδT cells than a blood preparation obtained by the method for producing an NK cell-enriched blood preparation according to the prior application, even if collected blood used is not only healthy individual-derived blood but blood derived from a patient having a disease such as cancer, which is an actual individual to be treated that should receive the NK cell-enriched blood preparation of the present invention.

All publications, patents, and patent applications cited herein are incorporated herein by reference in their entirety.

## Claims

1. A method for producing an NK cell-enriched blood preparation, comprising:
a stimulation step of stimulating NK cells comprised in blood collected from an organism, with NK cell growth-stimulating factors comprising at least an anti-CD 16 antibody, OK432, an anti-CD3 antibody, and a cytokine; and
a culture step of culturing the blood at a physiological cell temperature after the stimulation step.

2. The production method according to claim 1, wherein the stimulation step further comprises keeping the NK cells at 38°C to 40°C for 10 hours to 30 hours to apply thereto high-temperature stimulation.

3. The production method according to claim 1 or 2, wherein the anti-CD3 antibody is muromonab-CD3.

4. The production method according to any one of claims 1 to 3, wherein the cytokine is IL-2.

5. The production method according to any one of claims 1 to 4, wherein the NK cell growth-stimulating factors further comprise a bisphosphonate derivative or a salt thereof, or a hydrate thereof.

6. The production method according to claim 5, wherein the bisphosphonate derivative is selected from the group consisting of zoledronic acid, pamidronic acid, alendronic acid, risedronic acid, ibandronic acid, incadronic acid, etidronic acid, and a combination thereof.

7. The production method according to any one of claims 1 to 6, wherein the anti-CD16 antibody is immobilized on a solid-phase support.

8. The production method according to any one of claims 1 to 7, wherein the culture period in the culture step is 7 days to 21 days.

9. The production method according to any one of claims 1 to 8, wherein the blood is peripheral blood, cord blood, or bone marrow fluid.

10. An NK cell-enriched blood preparation obtained by a production method according to any one of claims 1 to 9.

11. A composition for NK cell enrichment comprising an anti-CD16 antibody, OK432, an anti-CD3 antibody, and a cytokine.

12. The composition according to claim 11, wherein the anti-CD3 antibody is muromonab-CD3.

13. The composition according to claim 11 or 12, wherein the cytokine is IL-2.

14. The composition according to any one of claims 11 to 13, further comprising a bisphosphonate derivative or a salt thereof, or a hydrate thereof.

15. The composition according to claim 14, wherein the bisphosphonate derivative is selected from the group consisting of zoledronic acid, pamidronic acid, alendronic acid, risedronic acid, ibandronic acid, incadronic acid, etidronic acid, and a combination thereof.

16. A kit for production of NK cell-enriched blood comprising a composition for NK cell enrichment according to any one of claims 11 to 15.
